# EUROPEAN PATENT SPECIFICATION

(11) **EP 4 281 588 B1**
(45) Date of publication and mention of the grant of the patent: **21.05.2025**
(21) Application number: 22702433.8
(22) Date of filing: 24.01.2022
(51) Int. Cl.: C12Q 1/6886

(54) **A METHOD FOR DIAGNOSING ENDOMETRIAL OR OVARIAN CARCINOMA**
VERFAHREN ZUR DIAGNOSE VON ENDOMETRIUMKARZINOMEN UND OVARIALKARZINOMEN
PROCÉDÉ PERMETTANT DE DIAGNOSTIQUER LE CARCINOME ENDOMÉTRIAL OU LE CARCINOME OVARIEN

(30) Priority: 25.01.2021 EP 21305086; 28.09.2021 EP 21306344
(43) Date of publication of application: 29.11.2023
(73) Proprietor: Université Paris Cité, 75006 Paris (FR); Assistance Publique Hôpitaux de Paris, 75004 Paris (FR); Centre National de la Recherche Scientifique, 75016 Paris (FR); INSERM (Institut National de la Santé et de la Recherche Médicale), 75013 Paris (FR); SORBONNE UNIVERSITE, 75006 Paris (FR)
(72) Inventor: ALEXANDRE, Jérôme, 94210 Saint Maur des Fossés (FR); BORGHESE, Bruno, 75006 Paris (FR); BEINSE, Guillaume, 75012 Paris (FR); TALY, Valérie, 92340 Bourg-la-Reine (FR); LAURENT-PUIG, Pierre, 92190 Meudon (FR); JUST, Pierre-Alexandre, 91430 Igny (FR)
(74) Representative: Cabinet Becker et Associés
(86) International application number: PCT/EP2022/051512
(87) International publication number: WO 2022/157369

(56) References cited:
- WO-A1-2013/022872
- WO-A1-2017/201606
- JIANG SHI-WEN ET AL: "Application of DNA methylation biomarkers for endometrial cancer management", vol. 8, no. 5, 1 September 2008 (2008-09-01), GB, pages 607 - 616, XP055819978, ISSN: 1473-7159, Retrieved from the Internet <URL:https://www.ncbi.nlm.nih.gov/pmc/articles/PMC5650066/pdf/nihms350277.pdf> DOI: 10.1586/14737159.8.5.607
- OLKHOV-MITSEL EKATERINA ET AL: "Epigenome-Wide DNA Methylation Profiling Identifies Differential Methylation Biomarkers in High-Grade Bladder Cancer", TRANSLATIONAL ONCOLOGY,, vol. 10, no. 2, 1 April 2017 (2017-04-01), pages 168 - 177, XP002782348, DOI: 10.1016/J.TRANON.2017.01.001
- MOARII MATAHI ET AL: "Epigenomic Alterations in Breast Carcinoma from Primary Tumor to Locoregional Recurrences", vol. 9, no. 8, 6 August 2014 (2014-08-06), pages e103986, XP055819836, Retrieved from the Internet <URL:https://storage.googleapis.com/plos-corpus-prod/10.1371/journal.pone.0103986/1/pone.0103986.pdf?X-Goog-Algorithm=GOOG4-RSA-SHA256&X-Goog-Credential=wombat-sa@plos-prod.iam.gserviceaccount.com/20210630/auto/storage/goog4_request&X-Goog-Date=20210630T131306Z&X-Goog-Expires=86400&X-Goog-SignedHeaders=h> DOI: 10.1371/journal.pone.0103986

## Description

The disclosure relates to a method for detection of endometrial or ovarian carcinoma by detecting hypermethylation of selected genomic sites.

### Background of the invention

Ovarian and endometrial carcinomas comprise the majority of gynecological carcinomas in developed countries. They share many histological features (endometrioid, serous, clear cell histotypes) as well as molecular alterations (e.g. TP53 alteration in serous carcinoma). Endometrial carcinoma is the most incident pelvic gynecological cancer. Most patients (90%) are diagnosed with localized diseases (FIGO stages I-III) and treated by radical carcinologic surgery and radiation therapy. Around one third of them experience relapse and ultimately die of their disease. The PORTEC-3 randomized clinical trial, which included patients with high-risk endometrial carcinoma to assess the potential benefit of chemotherapy, showed only a small improvement of the overall survival (De Boer et al. 2019, Lancet Oncol; 20: 1273-85). Overall, adjuvant therapeutic strategies in endometrial carcinoma are limited by the lack of biomarkers that are predictive of relapse.

The Cancer Genome Atlas consortium analyses identified four molecular groups (Nature Genetics, 2013, 45:1113-1120): most aggressive tumors, bearing a high number of copy-number alterations (copy-number high, CNH), carrying TP53 mutations, account for 20-25% of stage I-III tumors, and 40-50% of stage IV tumors. On the other hand, the best prognosis is observed in 5 to 10% of tumors with POLE mutation (Soumerai et al. Clin Cancer Res, 2018, 24(23):5939-5947). These molecular groups were found to be associated with a risk of relapse. However, the clinical relevance of this classification is limited to the consideration of the POLE- and TP53-mutated molecular groups, the latter being the group in which adjuvant chemotherapy has been found to improve prognosis in ancillary analyses of the PORTEC-3 study (León-Castillo et al. 2020, J Clin Oncol, 38(29):3388-3397). Jiang Shi-Wenet al. (EXPERT REVIEWS IN MOLECULAR DIAGNOSTICS, vol. 8, no. 5, 1 September 2008, pages 607-616) discloses methylation biomarkers for endometrial cancer management.

Despite new advances in the molecular characterization of endometrial carcinoma and in the patient's prognostication, all these strategies are limited by the lack of consideration of residual micro-metastatic disease. Recent studies focused on circulating tumor DNA (ctDNA) but were limited to the detection of frequent mutations in patient plasma (Bolivar et al. 2019, Modern Pathology, 32(3):405-414, Shintani et al. 2020, Int J Gynecol Cancer, 30(9):1340-1346). There is still a need for methods for early diagnosis and accurate monitoring of endometrial or ovarian carcinoma.

### Summary of the invention

The invention is defined by the claims. Any subject-matter falling outside the scope of the claims is provided for information purposes only.

The inventors have now identified a universal methylation signature of endometrial or ovarian carcinoma.

More particularly a subject of the invention is an *in vitro* method for detecting or monitoring an endometrial or ovarian carcinoma in a human subject, which method comprises detecting, or determining the level of, methylation of OXT and/or ZSCAN12 genes in a biological sample from the subject, wherein the biological sample contains genomic DNA.

Detecting, or determining the level of, methylation of OXT and ZSCAN12 genes in a biological sample can also make it possible to discriminate between an endometrial or an ovarian carcinoma.

In a particular embodiment, the method for detecting or monitoring an endometrial carcinoma comprises detecting, or determining the level of, methylation of OXT and ZSCAN12 gene in the biological sample.

In another particular embodiment, the method for detecting or monitoring an ovarian carcinoma comprises detecting, or determining the level of, methylation of OXT gene in the biological sample.

As explained in greater details below, the method advantageously comprises determining the methylation status of one or more CpG dinucleotides in a region of said genomic DNA that consists of chr20:3071846-3072247 (Hg38 coordinates) and/or chr6:28399594-28399995 (Hg38 coordinates), preferably chr20:3071946-3072147 (Hg38 coordinates) and/or chr6:28399694-28399895 (Hg38 coordinates).

More particularly, a hypermethylation at the selected genomic sites is indicative of an endometrial or ovarian carcinoma. In a preferred aspect, the sample is plasma and the hypermethylation at the selected genomic sites is indicative of the circulation of endometrial carcinoma or ovarian -originated DNA.

In a preferred embodiment, the sample contains ctDNA. ctDNA is a component of circulating cell-free DNA that is shed by malignant tumors into the bloodstream and other bodily fluids. Levels of ctDNA are typically low, particularly in patients with localized disease. By identifying hypermethylation at the selected genomic sites of the ctDNA, the invention allows for easy detection and assessment of earlier stages of disease, post-treatment molecular residual disease (MRD), resistance to targeted systemic therapy, and tumor mass burden.

### Legends to the drawing

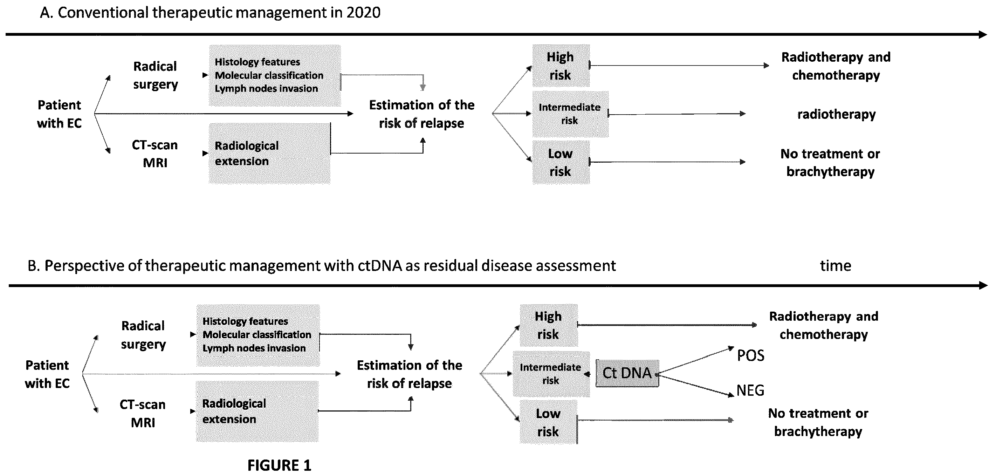

Figure 1 shows conventional (A) and potential (B) therapeutic work flow for endometrial carcinoma patients. EC: endometrial carcinoma. CT: computer tomography. MRI: magnetic resonance imaging. Nodes refer to lymph node. Brachytherapy: vaginal internal radiotherapy.

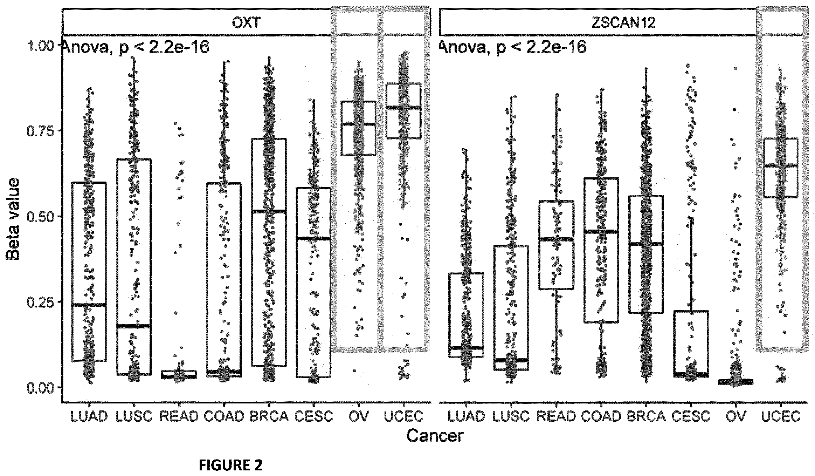

Figure 2 shows methylation levels of OXT and ZNCAN12 in endometrial carcinoma (435 subjects) and ovarian carcinoma (601 subjects), compared to most frequent cancers in women (lung adenocarcinoma (LUAD, 481 subjects) and squamous cell carcinoma (LUSC, 372 subjects), rectal (READ, 99 subjects) and colon adenocarcinoma (COAD, 317 subjects), breast cancer (BRCA, 798 subjects), and cervical squamous cell carcinoma (CESC, 312 subjects).

### Detailed description of the invention:

### Endometrial carcinoma

The methods of the invention encompass diagnosing and/or monitoring endometrial carcinoma in a subject. All histologic types, stages and grades are encompassed.

Endometrial carcinoma, also called endometrial cancer, starts in the cells of the epithelial inner lining of the uterus, i.e. the endometrium. Endometrial carcinomas can be divided into different histologic types. They include endometrioid adenocarcinoma, serous carcinoma, uterine carcinosarcoma (also known as mixed Mullerian tumors), clear-cell carcinoma, and rarely squamous cell carcinoma, small cell carcinoma, or transitional carcinoma.

Endometrial carcinoma stagging is summarized by the International Federation of Gynecology and Obstetrics (FIGO) 2010 classification. As used herein, the term "stage I" refers to endometrial cancer that is confined to the uterus, the term "stage II" refers to endometrial cancer that has spread from the uterus corpus to the uterus cervix (the lower part of the uterus) but has not spread outside the uterus, the term "stage III" refers to endometrial cancer that has spread to one or more of the following: the outermost layer of the uterus (uterine serosa), the tissue just beyond the uterus (i.e. adnexa-tissues on either side of the uterus) or the ovary.

An "early" diagnosis or detection means that the endometrial carcinoma is a stage I cancer.

As used herein, the term "FIGO grading" refers to the pathological grading system for carcinoma of the endometrium . This system is required for carcinoma of the endometrium under the College of American Pathologists' protocol. Grading excludes serous or clear cells, which are considered high grade (grade 3). Alternative grading divides endometrioid tumors into low grade or high grade based on solid growth (50 percent or less vs. 50 percent +), pattern of invasion (infiltrative vs. expansive) and presence of tumor cell necrosis (yes vs. no). A patient will be diagnosed with high grade if the tumors cells have 2 of these features. As used herein, the term "grade 1" or "FIGO Grade 1" refers to endometrial cells that resemble microglandular hyperplasia and are composed primarily of well-formed glands, having <5 percent nonsquamous solid component. The term "grade 2" or "FIGO Grade 2" refers to endometrial cells that are composed of between 6 percent and 50 percent nonsquamous solid components. The term "grade 3" or "FIGO Grade 3" refers to endometrial cells that have more than 50 percent nonsquamous solid component, lack well-formed glands, which differentiates it from serous endometrial carcinoma.

### Ovarian carcinoma

The methods of the invention encompass diagnosing and/or monitoring ovarian carcinoma in a subject. All histologic types, stages and grades are encompassed.

Approximately 90% of primary malignant ovarian tumors are epithelial (carcinomas), and are thought to arise from the ovarian surface epithelium or from surface epithelial inclusion cysts. The classification of ovarian epithelial tumors currently used by pathologists is based entirely on tumor cell morphology. The four major types of epithelial tumors (serous, endometrioid, clear cell, and mucinous) bear strong resemblance to the normal cells lining different organs in the female genital tract.

Ovarian cancer stages range from stage I (1) through IV (4). Two systems may be used for staging ovarian cancer, the FIGO (International Federation of Gynecology and Obstetrics) system and the AJCC (American Joint Committee on Cancer) TNM staging system.

### The subjects

The "subject" or "patient" may be any female mammal, especially a woman.

In certain embodiments, the subject has already been diagnosed with endometrial carcinoma or ovarian carcinoma. The method of the invention is then particularly useful for monitoring the impact of a therapeutic treatment on the endometrial carcinoma or ovarian carcinoma and/or assessing post-treatment molecular residual disease (MRD). Such treatment may include surgery, radiation therapy, chemotherapy, targeted therapy, and/or immunotherapy.

The method of the invention is particularly useful to measure tumor residual disease before or after treatment, typically before or after each treatment in the localized or metastatic setting. This makes it possible to avoid or, to the contrary, to increase adjuvant treatments.

The method of the invention is thus useful in determining the risk of relapse in a subject who has been subjected to a therapeutic treatment for endometrial carcinoma or ovarian carcinoma.

**Figure** 1 shows an example of a potential decision tree after residual disease assessment.

In certain embodiments, the subject is at risk of developing an endometrial carcinoma or ovarian carcinoma. Subjects with genetic predisposition are particularly encompassed, e.g. subjects with Lynch syndrome. Other risk factors for endometrial carcinoma include
- having had breast or ovarian cancer in the past,
- having had endometrial hyperplasia in the past, or
- treatment with radiation therapy to the pelvis to treat another cancer

The method of the invention may then be useful for early diagnosis of an endometrial carcinoma or ovarian carcinoma.

In other embodiments, the method of the invention is also useful for an early diagnosis of a cancerous lesion in the endometrium or ovarian epithelium in a subject who has been developing metastasis from a primary tumor of unknown origin.

### The sample

The methods of the invention require a biological sample from the subject, wherein the biological sample contains genomic DNA.

The sample may be a body fluid, a tissue sample, or a combination thereof, from the subject. The sample may comprise cell-free DNA.

In a preferred embodiment, the sample is a body fluid, preferably selected from the group consisting of plasma, serum, blood, or urine. Still preferably the sample is a plasma or serum sample and the DNA is circulating cell-free DNA (ccfDNA). In another embodiment, the sample may be ascitic or peritoneal fluid, uterine flushing, or uterine aspirate.

In another embodiment, the sample is a tissue sample. For instance, the sample may be obtained from an endometrial tissue. e.g. a pelvic, endometrial, or hysteroscopic biopsy. Endometrial tissues or cells may be taken, for example, by scrapes, smears, or swabs. Other means include punch biopsy, endocervical curettage, conization, resection of tumor samples, tissue samples prepared by endoscopic means, needle-biopsies of organs.

In another embodiment, the sample may be obtained from an ovarian tissue, e.g. resection of tumor samples, tissue samples prepared by endoscopic means, needle-biopsies of organs.

After collection, samples are prepared prior to detection of biomarkers. Sample preparation includes isolation of nucleic acids. These isolation procedures involve separation of nucleic acids from insoluble components (e.g., cytoskeleton) and cellular membranes. Typically, endometrial or ovarian tissues or cells may be treated with a lysis buffer solution prior to isolation of nucleic acids. A lysis buffer solution is designed to lyse tissues, cells, lipids and other biomolecules potentially present in the raw tissue samples. Nucleic acids can be conveniently extracted from biological samples, e.g. obtained from endometrium (i.e., endometrial tissues) or from ovarian tissues, using standard extraction methods that are known in the art. Standard extraction methods include the use of a chemical agent such as guanidinium thiocyanate, phenol-chloroform extraction, guanidine-based extraction, and the like. Commercial nucleic acid extraction kits may be employed.

### Methylation status:

According to the invention, the methylation status of the target genes serves as a biomarker for endometrial or ovarian carcinoma. CpG islands at the selected genomic sites are more particularly targeted.

Methylation of cytosine (C) nucleotides in CpG dinucleotide sequences (CpG sites; G= guanosine) of the human DNA is a known phenomenon. Moreover, abnormal methylation (hypermethylation and/or hypomethylation) of specific genomic areas is present in virtually all cancer types. Genomic regions with altered methylation states in test samples compared to controls samples are commonly referred to as "differentially methylated regions" (DMRs).

A "CpG island" as used herein refers to regions of DNA with a high G/C content and a high frequency of CpG dinucleotides relative to the whole genome. Also used interchangeably in the art is the term "CG island." The 'p' in "CpG island" refers to the phosphodiester bond between the cytosine and guanine nucleotides. Methylation may refer to methylation and/or hydroxymethylation of DNA.

Methods to determine methylation status of genomic DNA are well known to the skilled person, for example by bisulphite sequencing or related techniques such as: methylation specific PCR, microarray analysis of bisulphite converted DNA, pyrosequencing methylation assay.

In a preferred aspect of the invention, the detecting of methylation is performed by employing a digital PCR, preferably a digital droplet PCR (ddPCR).

As used herein, "digital PCR" refers to an assay that provides an end-point measurement that provides the ability to quantify nucleic acids without the use of standard curves, as is used in real-time PCR. Digital PCR includes a variety of formats, including employing droplet digital PCR, BEAMing (beads, emulsion, amplification, and magnetic), microwell plates, bulk emulsion droplets, microfluidic compartments and nanoliter- or picoliter-scale droplets produced with microfluidics.

"Droplet digital PCR" (ddPCR) refers to a digital PCR assay that measures absolute quantities by counting nucleic acid molecules encapsulated in discrete, volumetrically defined, water-in-oil droplet partitions that support PCR amplification (Hindson, et al, 2011. Analytical Chemistry 83, 8604-8610; Pekin, et al, 2011, Lab on a Chip 11, 2156-66; Pinheiro, et al, 2012, Anal Chem 84, 1003-1011). A single ddPCR reaction may be comprised of at least 13,000-20,000 partitioned droplets per well.

Upon evaluating a methylation state, the methylation state is often expressed as the fraction or percentage of individual strands of DNA that is methylated at a particular site (e.g., at a single nucleotide, at a particular region or locus, at a longer sequence of interest, e.g., up to a ~100-bp, 200-bp, subsequence of a DNA) relative to the total population of DNA in the sample comprising that particular site. Traditionally, the amount of the unmethylated nucleic acid is determined by PCR using calibrators. In a particular embodiment, a known amount of DNA is e.g. bisulfite treated and the resulting methylation-specific sequence is determined using any technique of exponential amplification.

In certain embodiments, the technology provides methods for characterizing a sample obtained from a human subject, the method comprising reacting a nucleic acid comprising a DMR with a reagent capable of modifying DNA in a methylation- specific manner (e.g., a methylation-sensitive restriction enzyme, a methylation-dependent restriction enzyme, and a bisulfite reagent) to produce nucleic acid modified in a methylation- specific manner; sequencing the nucleic acid modified in a methylation-specific manner to provide a nucleotide sequence of the nucleic acid modified in a methylation-specific manner; comparing the nucleotide sequence of the nucleic acid modified in a methylation-specific manner with a nucleotide sequence of a nucleic acid comprising the DMR from a subject who does not have endometrial or ovarian carcinoma to identify differences in the two sequences.

In certain embodiments, the technology provides systems for characterizing a sample obtained from a human subject, the system comprising an analysis component configured to determine the methylation state of a sample, a software component configured to compare the methylation state of the sample with a control sample or a reference sample methylation state recorded in a database, and an alert component configured to determine a single value based on a combination of methylation states and alert a user of a endometrial or ovarian carcinoma-associated methylation state. In some embodiments, the sample comprises a nucleic acid comprising a DMR.

In some embodiments, such systems further comprise a component for isolating a nucleic acid. In some embodiments, such systems further comprise a component for collecting a sample.

### The target genes

The term "gene" refers to a nucleic acid sequence that comprises coding sequences necessary for the production of an RNA, or of a polypeptide or its precursor. The term "gene" encompasses the coding regions of a structural gene and includes sequences located adjacent to the coding region on both the 5' and 3' ends, e.g., for a distance of about 1 kb on either end, such that the gene corresponds to the length of the full- length mRNA, e.g., comprising coding, untranslated regions, structural and other sequences and regulatory sequences, e.g. promoters. The sequences that are located 5' of the coding region and that are present on the mRNA are referred to as 5' non-translated or untranslated sequences. The sequences that are located 3' or downstream of the coding region and that are present on the mRNA are referred to as 3' non-translated or 3' untranslated sequences. The term "gene" in the present invention more particularly refers to genomic forms of a gene. In addition to containing introns, genomic forms of a gene may also include sequences located on both the 5' and 3' ends of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, posttranscriptional cleavage, and polyadenylation.

The human gene for oxytocin-neurophysin I (OXT) is physically mapped to chromosome 20p13. Preferably, the present methods comprise determining the methylation status of one or more CpG dinucleotides in the promoter region of the OXT gene. More particularly, the methylation status of one or more CpG dinucleotides is determined with respect to a genomic DNA sequence that comprises or consists of SEQ ID NO:1, or of a polymorphic variant sequence that has at least 90% sequence identity, preferably at least 95%, still preferably at least 98%, with SEQ ID NO:1.

SEQ ID NO:1:
TTTTCGAGCGAGTTTTAGCGTCGTTTCGGTTTTCGTTTATTCGTTCGTTTTCGT
AGTGTTTTTTTGCGGTTTCGGGGTAAAGGTCG

SEQ ID NO: 1 corresponds to the selected amplicon which targets a sequence targeted by the Illumina HM450 array probe cg06404175 on position chr20:3072046 (extended to +/-200 bp or +/-100 bp, e.g. chr20:3071946-3072147 (Hg38 coordinates)).

The ZSCAN12 gene (Zinc finger and SCAN domain-containing protein 12) maps on human chromosome 6, at 6p21.

Preferably, the present methods comprise determining the methylation status of one or more CpG dinucleotides in the promoter region of the ZSCAN12 gene. More particularly, the methylation status of one or more CpG dinucleotides is determined with respect to a genomic DNA sequence that comprises or consists of SEQ ID NO:2, or of a polymorphic variant sequence that has at least 90% sequence identity, preferably at least 95%, still preferably at least 98%, with SEQ ID NO:2.

SEQ ID NO:2:
ATAAAGGTCGGAAGCGGTTACGGGGGGTTTAAGAATTAGTTCGCGCGGGGCGT
ATTTTCGCGGTCGTTTTAGGAAGGGAGCGAAAGG

SEQ ID NO: 2 corresponds to the selected amplicon which targets a sequence targeted by the Illumina HM450 array probe cg25060829 on position chr6:28399794 (extended to +/-200 bp or +/-100bp, e.g. chr6:28399694-28399895 (Hg38 coordinates)).

In a particular embodiment, the method of the invention comprises determining the methylation status of the DNA; and comparing the methylation status of the DNA to one or more methylated probes selected from the group consisting of cg06404175 and cg25060829, or preferably both, wherein the methylated probe comprises a sequence region that optionally extends up to 250 base pairs, preferably less than 150 bp, still preferably less than 80bp, upstream and downstream from the methylated probe, and wherein the comparison indicates whether the sample is unmethylated or not at this genomic locus, i.e. indicative of the presence of an endometrial carcinoma.

In another particular embodiment, the method of the invention comprises determining the methylation status of the DNA; and comparing the methylation status of the DNA to methylated probe cg06404175, wherein the methylated probe comprises a sequence region that optionally extends up to 250 base pairs, preferably less than 150 bp, still preferably less than 80bp, upstream and downstream from the methylated probe, and wherein the comparison indicates whether the sample is unmethylated or not at this genomic locus, i.e. indicative of the presence of an ovarian carcinoma.

Kits are also provided which comprise probes and/or primers, and optionally additional reagents, useful for determining the methylation status at the genomic sites described above.

### Selection of treatments

It is further disclosed a method for identifying or screening a therapeutic agent or candidate therapeutic agent, for use in treating an endometrial carcinoma or an ovarian carcinoma in a subject, which method comprises administering the subject with said therapeutic agent and detecting, or determining the level of, methylation of OXT and/or ZSCAN12 gene in a biological sample from the subject, wherein the biological sample contains genomic DNA, wherein a decreased of methylation after administration of the therapeutic agent is indicative of an agent that has a benefit on the endometrial or ovarian carcinoma in the subject.

The therapeutic agent may be a chemotherapeutic or immunotherapeutic agent, such as e.g. Paclitaxel (Taxol^{®}), Carboplatin, Doxorubicin (Adriamycin^{®}) or liposomal doxorubicin (Doxil^{®}/Caelyx^{®}), Cisplatin, Gemcitabine, Docetaxel (Taxotere^{®}), or combinations thereof, as well as ifosfamide (Ifex^{®}) or targeted therapies such as anti-HER2 antibodies such as trastuzumab (Herceptin^{®}), PARP inhibitors (e.g. Olaparib, Niraparib, Talazoparib), angiogenesis inhibitors (e.g. Nindetanib).

Further disclosed is a method for treating a subject that has been diagnosed with an endometrial or ovarian carcinoma using the method of diagnosis of the invention, wherein the subject is then administered with an effective amount of a therapeutic agent, including chemotherapy and/or immunotherapy, or is subjected to surgery and/or radiation.

The Examples and Figures illustrate the invention without limiting its scope.

### Example 1 : Identification of hypermethylated gene candidates

### 1.1.In silico preliminary analysis

Two *in silico* approaches were chosen to identify candidate CpG positions.

Firstly a machine-learning approach was used to learn a model predictive of the tumor versus non-tumor nature of an endometrial sample (LASSO-penalized logistic regression; R package glmnet).

Secondly a differential methylated position analysis selected most differentially methylated positions between endometrial carcinoma and (i) non-cancer samples (uterine and non-uterine), (ii) blood samples. Position commonly differentially methylated were then selected. Among these, the inventors manually selected only CpG positions with (i) no more than 5% of non-tumor sample with beta value > 0.1, and (ii) more than 75% of tumor sample with beta value > 0.6.

These two in silico analyses led to the selection of three targets have been found to be highly methylated in endometrial cancer and not methylated in other tissues: the positions referred to SIM1, OXT and ZSCAN12. Among these, SIM1 showed low methylation levels in white blood cells methylation analyses and was therefore not selected for further analyses.

### 1.2.Confirmation of the hypermethylation of OXT and ZSCAN12 as a universal signature in endometrial carcinoma

A first control allowed to confirm whether the positions found to be hypermethylated are unequivocally methylated, i.e. neighbor positions in the Illumina HM450 array are detected as consistently hypermethylated in endometrial carcinoma samples. For this purpose, all Illumina HM450 array positions overlapping the 5000 bp in up and downstream of OXT and ZSCAN12 transcription starting sites (consensus transcript between RefSeq and Ensembl data base) were selected using the GenomicRanges R functions.

A second control allowed to confirm whether the positions found to be hypermethylated are hypermethylated regardless of endometrial carcinoma molecular subgroups. For this purpose, the methylation levels of the selected targeted positions were compared across endometrial carcinoma TCGA molecular subgroups. Data used were publicly available TCGA data Considering both OXT and ZSCAN12-associated methylated positions, only 1.5% of samples showed double-hypomethylation.

A third control allowed to confirm whether the positions found to be hypermethylated were also methylated in metastatic samples. Indeed, genomic modifications can occur during the metastatic process, and could lead to DNA methylation modifications. For this purpose, the methylation level of the selected targeted positions was analyzed in metastatic samples previously published (data publicly available at https://www.ncbi.nlm.nih.gov/geo/. Positions OXT and/or ZSCAN12 showed high methylation value (>0.25) in all metastatic samples.

### Example 2 : Evaluation of the diagnostic performance of the endometrial carcinoma signature

The positions referring to OXT and ZSCAN12 were thus selected for a ddPCR assay development targeting hypermethylated template DNA that was previously bisulfite-treated. Albumine (ALB) was used as control.

### Methods

### Bisulfite conversion of DNA

Up to 20µL (or 30 ng for highly concentrated DNA samples) of DNA samples extracted from plasma, buffy coat, or tumors, were bisulfite-converted using the EZ DNA Methylation-Gold Kit (ZYMO research), according to the manufacturer's instructions. Bisulfite-converted DNA was stored up to one week at -20°C until ddPCR. Bisulfite-converted DNA were eluted in M-elution buffer 11µL. Various amounts of human genomic control DNA and universally methylated human DNA were bisulfite-converted depending on the experiment (development steps or as positive control in each ddPCR experiment). Every bisulfite-conversion batch included at least one well with 10 ng of human genomic DNA control and one well with 10 ng of universally methylated human DNA control.

### ddPCR assay

Technical performances of the ddPCR assay were assessed using guidelines for ddPCR assay development (The dMIQE Group, 2020, Clinical Chemistry, 66(8):1012-1029).

For each target, we designed forward and reverse primers (Eurofins Genomics, France), and a MGB Taqman probe^{®} (Applied BiosystemsTM, UK) aiming to amplify and detect methylated sequences of the targeted amplicons identified by the bioinformatical analysis. A methylation-insensitive target on the gene of ALB (encoding albumin) was used as an internal control of the amount of DNA analyzed in each PCR well (primers and probes reported below).

| **Target** | **Reporter dve** | **Probe Sequence (5'>3')** | **Concentration in PCR reaction (µM)** |
|---|---|---|---|
| ZSCAN12 | FAM | TTAAGAATTAGTT**CGCGCG**G (SEQ ID NO:3) | 0.4 |
| OXT | FAM/VIC | T**CG**GTTTT**CG**TTTATTT**CG**TT**CG** (SEQ ID NO:4) | 0.2 / 0.2 |
| ALB | VIC | AGGGTTTTTATAATTTA (SEQ ID NO:5) | 0.6 |

| **Target** | **Forward Primer sequence (5'>3')** | **Reverse Primer sequence (5'>3')** |
|---|---|---|
| | **(0.4µM)** | **(0.4µM)** |
| ZSCAN12 | ATAAAGGT**CG**GAAG**CG**GTTA (SEQ ID NO:6) | CCTTT**CG**CTCCCTTCCTAAA (SEQ ID NO:7) |
| OXT | TTTT**CG**AG**CG**AGTTTTTAGCG (SEQ ID NO:8) | **CG**ACCTTTACCCC**CG**AAAC (SEQ ID NO:9) |
| ALB | GGGATGGAAAGAATTTTATGTT (SEQ ID NO:10) | AAACAAACTAACCCCAAATTCT (SEQ ID NO:11) |

| **Step** | **Temperature** | **Time** | **Ramp rate** | |
|---|---|---|---|---|
| **I. Enzyme activation** | 95°C | 10 min | 2.5°/s | |
| **II. DNA denaturation** | 94°C | 30 s | 2.5°/s | |
| **III. Annealing and extension** | 58.4°C | 1 min | 2.5°/s | Return to step II x 44 times |
| **IV. Inactivation step** | 98°C | 10 min | | |
| **V. Hold** | 12°C | Forever | | |

The theoretical limit of blank (LOB) and limit of detection (LOD) were assessed by running the assay on more than 30 replicates of negative control (bisulfite-treated genomic DNA extracted from pool of whole blood lysate (G304, PromegaTM, USA), and by calculation using previously established procedures (Armbruster & Pry 2008, Clin Biochem Rev, 29 Suppl 1, S49-52). LOB:LOD (droplet/20µL:cp/20µL) were 2:6 and 2:6, for ZSCAN12 and OXT, respectively. Technical sensitivity within each well was assessed by applying the ddPCR assay on log10 serial dilution of hypermethylated DNA (D5011-1, Zymo Research TM, USA) and estimated to 0.1% (detection of 0.01 ng of methylated DNA diluted in 10 ng of human genomic DNA).

Target hypermethylation in endometrial carcinoma and non-cancer samples. *one tumor-adjacent tissue had in situ carcinoma infiltration
The diagnostic performances of the universal signature of methylation was assessed in an independent cohort of 78 patients followed at Cochin hospital, previously included in a study aiming to characterize the molecular landscape of their tumors (Beinse et al. Plos One 2019, 14(3):e0214416; Beinse et al. 2020, Int J Gynecol Cancer, 30(5):640-647). With a sensitivity of 99% and a specificity of for detection of 93%, this signature allowed to accurately identify all but one tumor sample. Non-tumor samples (N=28) being tissues adjacent to tumors, the specificity could be underestimated. See Table below.

| | **Tumor samples N = 79** | **Normal samples N=28** |
|---|---|---|
| Samples with ZSCAN12 and/or OXT methylation ratio/ALB >0.05 | 78 (one CIS) | 2 |
| Unmethylated samples | 1 | 26 |

The specificity of the universal methylation signature was assessed by applying the ddPCR assay on 35 samples of DNA extracted from 35 plasma samples (QIAamp^{®} Circulating Nucleic Acid kit, QIAGEN^{®}). Among these, 5 samples were obtained from healthy donors (Biopredic International, France), and 30 were obtained from patients treated for non-neoplastic gynecologic conditions at Cochin Hospital. No sample showed any positive droplet detecting hypermethylated sequences of ZSCAN12 or OXT.

### Example 3 : Investigation in other cancers

Figure 2 shows that the OXT/ZSCAN12 combination allows is a specific signature for endometrial carcinoma, compared to other cancers. The inventors have further shown that the OXT hypermethylation was a signature for ovarian carcinoma as well. Hence, in the TCGA database, 98% of ovarian cancer samples showed OXT hypermethylation.

When considering the methylation value of OXT and/or ZSCAN12, this combination allowed to discriminate endometrial and ovarian cancers (gathered) from all other cancers with an AUC of 0.84 (sensitivity = 0.98; specificity = 0.31)

## Claims

1. An *in vitro* method for detecting or monitoring an endometrial carcinoma in a human subject, which method comprises detecting, or determining the level of, methylation of OXT and/or ZSCAN12 gene in a biological sample from the subject, wherein the biological sample contains genomic DNA.

2. The method of claim 1, for detecting or monitoring an endometrial carcinoma, which comprises detecting, or determining the level of, methylation of OXT and ZSCAN12 gene in the biological sample.

3. The method of claim 1, comprising determining the methylation status of one or more CpG dinucleotides in a region of said genomic DNA that consists of chr20:3071846-3072247 (Hg38 coordinates) and/or chr6:28399594-28399995 (Hg38 coordinates), preferably chr20:3071946-3072147 (Hg38 coordinates) and/or chr6:28399694-28399895 (Hg38 coordinates).

4. The method of claim 1 or 2, comprising determining the methylation status of one or more CpG dinucleotides in a region of said genomic DNA that comprises or consists of sequence SEQ ID NO:1 and/or SEQ ID NO:2, or of a polymorphic variant sequence that has at least 90% sequence identity with SEQ ID NO:2 and/or SEQ ID NO:2.

5. The method according to any of claims 1 to 4, wherein the sample is a body fluid, preferably selected from the group consisting of plasma, serum, blood, and urine, still preferably wherein the sample is a plasma or serum sample and the DNA is circulating cell-free DNA (ccfDNA), preferably circulating tumor DNA (ctDNA).

6. The method according to any of claims 1 to 5, wherein the sample is a tissue sample, e.g. a pelvic biopsy.

7. The method according to any of claims 1 to 6, wherein the detecting of methylation is performed by employing a digital PCR, preferably a digital droplet PCR (ddPCR).

8. The method according to any of claims 1 to 7, for monitoring the impact of a therapeutic treatment on the carcinoma.

9. The method according to any of claims 1 to 7, for an early diagnosis in a subject who is at risk of developing said carcinoma.

10. The method according to any of claims 1 to 7, for an early diagnosis of a cancerous lesion in the endometrium epithelium in a subject who has been developing metastasis from a primary tumor of unknown origin.

11. The method according to any of claims 1 to 7, for assessing the risk of relapse.

12. The method according to claim 11, for assessing molecular residual disease (MRD), especially after treatment.

13. The method according to claim 11, for assessing the therapeutic resistance in patients with metastasis.

## Patentansprüche

1. In-vitro-Verfahrenzum Nachweis oder zur Überwachung eines Endometriumkarzinoms bei einem menschlichen Subjekt, wobei das Verfahren das Nachweisen oder das Bestimmen des Niveaus der Methylierung des OXT- und/oder ZSCAN12-Gens in einer biologischen Probe des Subjekts umfasst, wobei die biologische Probe genomische DNA enthält.

2. Das Verfahren nach Anspruch 1 zum Nachweis oder zur Überwachung eines Endometriumkarzinoms, das das Nachweisen oder das Bestimmen des Niveaus der Methylierung des OXT- und/oder ZSCAN12-Gens in der biologischen Probe umfasst.

3. Das Verfahren nach Anspruch 1, das das Nachweisen des Methylierungsstatus eines oder mehrerer CpG-Dinukleotide in einem Bereich dieser genomischen DNA umfasst, der aus chr20:3071846-3072247 (Hg38-Koordinaten) und/oder chr6:28399594-28399995 (Hg38-Koordinaten) besteht, vorzugsweise chr20:3071946-3072147 (Hg38-Koordinaten) und/oder chr6:28399694-28399895 (Hg38-Koordinaten).

4. Das Verfahren nach Anspruch 1 oder 2, das das Nachweisen des Methylierungsstatus eines oder mehrerer CpG-Dinukleotide in einem Bereich dieser genomischen DNA umfasst, der die Sequenz SEQ ID NO:1 und/oder SEQ ID NO:2 oder eine polymorphe Variantensequenz, die mindestens 90 % Sequenzidentität mit SEQ ID NO:2 und/oder SEQ ID NO:2 aufweist; umfasst oder daraus besteht.

5. Das Verfahren nach einem der Ansprüche 1 bis 4, wobei die Probe eine Körperflüssigkeit ist, vorzugsweise ausgewählt aus der Gruppe bestehend aus Plasma, Serum, Blut und Urin, wobei die Probe noch bevorzugter eine Plasma- oder Serumprobe ist und die DNA zirkulierende zellfreie DNA (ccfDNA), vorzugsweise zirkulierende Tumor-DNA (ctDNA), ist.

6. Das Verfahren nach einem der Ansprüche 1 bis 5, wobei die Probe eine Gewebeprobe ist, z. B. eine Beckenbiopsie.

7. Das Verfahren nach einem der Ansprüche 1 bis 6, wobei der Methylierungsnachweis durch Einsatz einer digitalen PCR, vorzugsweise einer digitalen Droplet-PCR (ddPCR), durchgeführt wird.

8. Das Verfahren nach einem der Ansprüche 1 bis 7 zur Überwachung der Auswirkungen einer therapeutischen Behandlung auf das Karzinom.

9. Das Verfahren nach einem der Ansprüche 1 bis 7 für eine Frühdiagnose bei einem Subjekt, bei dem das Risiko besteht, an dem Karzinom zu erkranken.

10. Das Verfahren nach einem der Ansprüche 1 bis 7 zur Frühdiagnose einer krebsartigen Läsion im Endometriumepithel bei einem Subjekt, bei dem sich Metastasen eines Primärtumors unbekannter Herkunft entwickelt haben.

11. Das Verfahren nach einem der Ansprüche 1 bis 7 zur Beurteilung des Rückfallrisikos.

12. Das Verfahren nach Anspruch 11 zur Beurteilung einer molekularen Resterkrankung (MRD), insbesondere nach einer Behandlung.

13. Das Verfahren nach Anspruch 11 zur Beurteilung der Therapieresistenz bei Patienten mit Metastasierung.

## Revendications

1. Méthode *in vitro* pour détecter ou surveiller un carcinome endométrial chez un sujet humain, laquelle méthode comprend la détection ou la détermination du niveau de méthylation des gènes OXT et/ou ZSCAN12 dans un échantillon biologique provenant du sujet, dans laquelle l'échantillon biologique contient de l'ADN génomique.

2. Méthode selon la revendication 1, pour détecter ou surveiller un carcinome endométrial, qui comprend la détection ou la détermination du niveau de méthylation des gènes OXT et ZSCAN12 dans l'échantillon biologique.

3. Méthode selon la revendication 1, comprenant la détermination du statut de méthylation d'un ou plusieurs dinucléotides CpG dans une région dudit ADN génomique qui consiste en chr20:3071846-3072247 (coordonnées Hg38) et/ou chr6:28399594-28399995 (coordonnées Hg38), de préférence chr20:3071946-3072147 (coordonnées Hg38) et/ou chr6:28399694-28399895 (coordonnées Hg38).

4. Méthode selon la revendication 1 ou 2, comprenant la détermination du statut de méthylation d'un ou plusieurs dinucléotides CpG dans une région dudit ADN génomique qui comprend ou consiste en les séquences SEQ ID NO : 1 et/ou SEQ ID NO : 2, ou une séquence variante polymorphique qui a une identité de séquence d'au moins 90 % avec la SEQ ID NO : 2 et/ou la SEQ ID NO : 2.

5. Méthode selon l'une quelconque des revendications 1 à 4, dans laquelle l'échantillon est un fluide corporel, de préférence choisi dans le groupe constitué par le plasma, le sérum, le sang et l'urine, mieux encore dans laquelle l'échantillon est un échantillon de plasma ou de sérum et l'ADN est un ADN libre circulant (ADNccf), de préférence un ADN tumoral circulant (ADNct).

6. Méthode selon l'une quelconque des revendications 1 à 5, dans laquelle l'échantillon est un échantillon tissulaire, par exemple une biopsie pelvienne.

7. Méthode selon l'une quelconque des revendications 1 à 6, dans laquelle la détection de méthylation est effectuée par l'emploi d'une PCR digitale, de préférence d'une PCR digitale en gouttelettes (ddPCR).

8. Méthode selon l'une quelconque des revendications 1 à 7, pour surveiller l'impact d'un traitement thérapeutique sur le carcinome.

9. Méthode selon l'une quelconque des revendications 1 à 7, pour un diagnostic précoce chez un sujet qui présente un risque de développement dudit carcinome.

10. Méthode selon l'une quelconque des revendications 1 à 7, pour un diagnostic précoce d'une lésion cancéreuse dans l'épithélium endométrial chez un sujet qui a développé des métastases à partir d'une tumeur primaire d'origine inconnue.

11. Méthode selon l'une quelconque des revendications 1 à 7, pour déterminer le risque de rechute.

12. Méthode selon la revendication 11, pour déterminer une maladie résiduelle moléculaire (MRD), en particulier après traitement.

13. Méthode selon la revendication 11, pour évaluer la résistance thérapeutique chez des patients ayant des métastases.
